# EUROPÄISCHE PATENTSCHRIFT

(11) **EP 1 121 339 B1**
(45) Veröffentlichungstag und Bekanntmachung des Hinweises auf die Patenterteilung: **29.01.2003**
(21) Anmeldenummer: 99947458.8
(22) Anmeldetag: 05.10.1999
(51) Int. Cl.: C07C 39/16, C07C 37/70

(54) **VERFAHREN ZUR HERSTELLUNG VON BIS(4-HYDROXYARYL)ALKANEN**
METHOD FOR THE PRODUCTION OF BIS(4-HYDROXYARYL)ALKANES
PROCEDE DE PRODUCTION DE BIS-(4-HYDROXYARYL)ALCANES

(30) Priorität: 17.10.1998 DE 19848026
(43) Veröffentlichungstag der Anmeldung: 08.08.2001
(73) Patentinhaber: Bayer Aktiengesellschaft, 51368 Leverkusen (DE)
(72) Erfinder: KÜHLING, Steffen, B-9100 Sient Niklaas (BE); LANZE, Rolf, D-47804 Krefeld (DE); NEUMANN, Rainer, D-47803 Krefeld (DE); HEYDENREICH, Frieder, D-40593 Düsseldorf (DE); VAN OSSELAER, Tony, D-47800 Krefeld (DE)
(86) Internationale Anmeldenummer: EP9907358
(87) Internationale Veröffentlichungsnummer: WO00023410

(56) Entgegenhaltungen:
- US-A- 5 629 457

## Beschreibung

Die vorliegende Erfindung betrifft ein Verfahren zur Herstellung von Bis(4-hydroxyaryl)alkanen und aromatischen Hydroxyverbindungen, die durch sauer katalysierte Umsetzung der aromatischen Hydroxyverbindungen mit Ketonen erhalten werden.

Die Synthese von Bis(4-hydroxyaryl)alkanen durch sauer katalysierte Umsetzung aromatischer Hydroxyverbindungen mit Ketonen ist bekannt, beispielsweise aus US-A 2 775 620 oder EP-A 342 758. In der Regel wird als Zwischenprodukt ein Addukt des Bis(4-hydroxyaryl)alkans und der als Edukt eingesetzten aromatischen Hydroxyverbindungerhalten, das anschließend destillativ von der aromatischen Hydroxyverbindung befreit wird. Das großtechnisch wichtigste Beispiel ist die Herstellung von Bisphenol A, bei der intermediär ein Addukt von Bisphenol A (BPA) und Phenol erhalten wird. Auch nach Reinigung, beispielsweise durch Umkristallisation, enthält dieses Addukt aufgrund der sauer katalysierten Herstellung noch Spuren von Säure (ca. 5 bis 10·10⁻⁶ mol Säure/mol BPA). Diese Säurespuren führen bei der Abtrennung des Phenols vom Bisphenol A, die mit einer thermischen Belastung verbunden ist, zu teilweiser Zersetzung des Bisphenols und der Bildung von Nebenprodukten. Folge dieser Zersetzungsreaktionen sind die Verschlechterung der Reinheit und Farbqualität des Bisphenols. Dies wirkt sich auch auf die Qualität der aus den Bisphenolen hergestellten Produkte wie Epoxidharze, Polyester, Polyestercarbonate und Polycarbonate negativ aus, und zwar dergestalt, daß Farbprobleme, schlechte Lichtdurchlässigkeit transparenter Produkte oder Strippen in den Oberflächen von Formteilen aus diesen Endprodukten die Folge sind. Analoge Verhältnisse treten auch bei der Herstellung anderer Bis(4-hydroxyaryl)alkane auf.

Die Abtrennung der aromatischen Hydroxyverbindung, auch "Strippen" genannt, vom Bis(4-hydroxyaryl)alkan ist literaturbekannt und beispielsweise in EP-A 343 349 beschrieben. Hierbei wird mit Dampf in einer gepackten Kolonne bei 160 bis 200°C bei leicht reduziertem Druck die aromatische Hydroxyverbindung vom Bis(4-hydroxyaryl)alkan abgetrennt. Bekannt ist auch, z.B. aus US-PS 5,091,159, daß bei der Abtrennung der aromatischen Hydroxyverbindung vom Bis(4-hydroxyaryl)alkan thermische Zersetzungsreaktionen auftreten. Ein weiteres Verfahren ist in US-A-5 629 457 beschrieben.

Es wurde nun ein Verfahren gefunden, das die Zersetzungsreaktionen beim Strippen der aromatischen Hydroxyverbindung ("Monophenol") vom Bis(4-hydroxyaryl)-alkan stark zurückdrängt. Hierbei wird bei der Abtrennung des Monophenols vom Bis(4-hydroxyaryl)alkan mit Inertgas nach der Kondensation des Monophenols das im Kreis geführte Inertgas gereinigt, so daß die Schädigung des Bis(4-hydroxyaryl)alkans vermieden werden kann.

Die im erfindungsgemäßen Verfahren einsetzbaren Addukte von Bis(4-hydroxyaryl)alkanen und aromatischen Hydroxyverbindungen sind erhältlich durch Umsetzung von aromatischen Hydroxyverbindungen, die in p-Position nicht substituiert sind und keine Substituenten zweiter Ordnung wie Cyano-, Carboxy- oder Nitrogruppen enthalten, beispielsweise Phenol, o- und m-kresol, 2.6-Dimethylphenol, o-tert.-Butylphenol, 2-Methyl-6-tert.-butylphenol, o-Cyclohexylphenol, o-Phenyl-phenol, o-Isopropylphenol, 2-Methyl-6-cyclopentyl-phenol, o- und m-Chlorphenol, 2,3,6-Trimethylphenol, bevorzugt Phenol, o- und m-kresol, 2,6-Dimethylphenol, o-tert.-Butylphenol und o-Phenyl-phenol, besonders bevorzugt Phenol, und Ketonen mit wenigstens einer aliphatischen Gruppe an der Carbonylfunktion, beispielsweise Aceton, Methylethylketon, Methylpropylketon, Methylisopropylketon, Diethylketon, Acetophenon, Cyclohexanon, Cyclopentanon, Methyl-, Dimethyl- und Trimethylcyclohexanonen, die auch geminale Methylgruppen aufweisen können, z.B. 3,3-Dimethyl-5-methylcyclohexanon (Hydroisophoron), bevorzugt Aceton, Acetophenon, Cyclohexanon und dessen Methylgruppen tragende Homologe: besonders bevorzugt Aceton. Bevorzugt wird als Edukt das Addukt von Bisphenol A und Phenol eingesetzt.

Gegenstand der Erfindung ist somit ein Verfahren zur Herstellung von Bis(4-hydroxyaryl)alkanen aus Addukten von Bis(4-hydroxyaryl)alkanen und aromatischen Hydroxyverbindungen, bei dem
a) durch eine Schmelze eines Addukts aus einem Bis(4-hydroxyaryl)alkan und einer aromatischen Hydroxyverbindung bei 150°C bis 230°C ein Inertgas geleitet wird, wobei der Inertgasstrom aromatische Hydroxyverbindung aus der Schmelze entfernt,
b) die aromatische Hydroxyverbindung aus dem Inertgasstrom durch Kondensation entfernt wird,
c) der Inertgasstrom gereinigt, komprimiert und wieder in Schritt a) zurückgeführt wird.

Die Abtrennung des Monophenols vom Bis(4-hydroxyaryl)alkan aus der Schmelze des Addukts aus Bis(4-hydroxyaryl)alkan und aromatischer Hydroxyverbindung erfolgt bei Temperaturen zwischen 150°C bis 230°C, bevorzugt bei 170°C bis 210°C, indem das Monophenol durch Eintragen eines Inertgases (z.B. Stickstoff) ausgetrieben wird. Das Verhältnis von Gas zu Addukt-Menge liegt bevorzugt bei ca. 10 m³ bis 1000m³ pro t Addukt. Das Strippen kann gegebenenfalls durch Verringerung des Druckes in dem zur Abtrennung des Monophenols eingesetzten Aggregat erleichtert werden, erfolgt aber bevorzugt unter Normaldruck. Das Strippen des Monophenols mit dem Inertgas wird in bekannten Apparaten, also beispielsweise in einer gepackten, geflutet betriebenen Kolonne, durchgeführt. Das Monophenol wird beispielsweise durch Kondensieren an einem Wärmetauscher aus dem Inertgaskreislaufsystem entfernt.

Die Reinigung des im Kreis geführten Inertgases kann durch Überleiten über ein Festbettadsorbens (Aktivkohle, Zeolith etc.) erfolgen oder in einer bevorzugten Anwendungsform, indem das Inertgas intensiv gewaschen wird. Dieses Waschen kann beispielsweise in einem Gaswäscher erfolgen. In einer bevorzugten Ausführungsform wird bei der Kompression des Inertgases des Kreislaufstroms in einem Kompressor die dort eingesetzte Sperrflüssigkeit als Waschmedium genutzt.

Als Waschmedium wird eine schwach alkalische wäßrige Lösung benutzt. Der pH-Wert der verwendeten wäßrigen Lösung sollte im Bereich von 7 bis 12, bevorzugt 7,5 bis 11, ganz besonders bevorzugt 8 bis 10 liegen. Zur Herstellung einer solchen wäßrigen Lösung können alle basisch wirkenden Substanzen eingesetzt werden, bevorzugt sind Alkali- und Erdalkalihydroxide. In einer bevorzugten Ausführungsform wird der pH-Wert vor und nach dem Waschen kontrolliert, so daß Über- und Unterdosierungen bei der Reinigung des Inertgaskreislaufs vermieden werden. Der Inertgasstrom kann anschließend dann noch einer zweiten Wäsche zugeführt werden. Das Waschmedium ist dann bevorzugt pH-neutral.

Die nach dem erfindungsgemäßen Verfahren hergestellten Bis(4-hydroxyaryl)alkane zeichnen sich durch eine sehr gute Eigenfarbe sowie hohe Reinheit aus, insbesondere weisen sie geringe Gehalte an aromatischen Hydroxyverbindungen (<100 ppm, bevorzugt <50 ppm) und Zersetzungsprodukten (z.B. Isopropenylphenol, dimeres Isopropenylphenol) auf.

Aus den nach dem erfindungsgemäßen Verfahren hergestellten Bis(4-hydroxyaryl)-alkanen können Polymere wie Polycarbonate oder Epoxide mit geringer Eigenfarbe hergestellt werden.

### Beispiele

### Beispiel 1

Ein 1 t/h Schmelzegemisch BPA/Phenol (60/40 Gew.-%) wird in einen Desorber eingebracht. In den Desorber werden kontinuierlich 225 m³ Stickstoff/h (Kreislaufstrom) eingeleitet. Das Phenol wird vom Stickstoff aufgenommen und anschließend mittels eines Wärmetauschers kondensiert. Der Stickstoff wird anschließend einem Kompressor, dessen Sperrflüssigkeit mit stark verdünnter NaOH-Lösung (pH-Wert 10) beaufschlagt wird, zugeführt, verdichtet und in einem Gaswäscher, der mit VE-Wasser betrieben wird, eingeleitet. Der so behandelte Stickstoff wird jetzt wieder im Desorber zur Phenolstrippung eingesetzt. Man erhält dann eine phenolarme (Phenolwert 40 ppm) farbhelle BPA-Schmelze mit einer Schmelzefarbzahl von 8 Hazen.

### Vergleichsbeispiel 1

Wie Beispiel 1, nur wird auf die Wäsche des Stickstoffs ganz verzichtet. Man erhält dann eine BPA-Schmelze mit einer Schmelzefarbzahl von 17 Hazen. Der Phenolwert im BPA liegt bei 90 ppm, was auf Zersetzung hinweist.

### Beispiel 2

Wie Beispiel 1, nur wird zur Wäsche des Stickstoffs ein neutrales VE-Wasser (pH-Wert 6,9) eingesetzt. Man erhält dann eine BPA-Schmelze mit einer Schmelzefarbzahl von 14 Hazen. Der Phenolwert im BPA liegt bei 75 ppm, was noch auf Zersetzung hinweist.

### Beispiel 3

Wie Beispiel 1, nur wird zur Wäsche des Stickstoffs eine verdünnte NaOH-Lösung (pH-Wert 13) eingesetzt. Man erhält dann eine BPA-Schmelze mit einer Schmelzefarbzahl von 12 Hazen. Der Phenolwert im BPA liegt bei 60ppm, was noch auf Zersetzung hinweist.

## Patentansprüche

1. Verfahren zur Herstellung von Bis(4-hydroxyaryl)alkanen aus Addukten von Bis(4-hydroxyaryl)alkanen und aromatischen Hydroxyverbindungen, bei dem
a) durch eine Schmelze eines Addukts aus einem Bis(4-hydroxyaryl)alkan und einer aromatischen Hydroxyverbindung bei 150°C bis 230°C ein Inertgas geleitet wird, wobei der Inertgasstrom aromatische Hydroxyverbindung aus der Schmelze entfernt,
b) die aromatische Hydroxyverbindung aus dem Inertgasstrom durch Kondensation entfernt wird,
c) der Inertgasstrom gereinigt, komprimiert und wieder in Schritt a) zurückgeführt wird.

2. Verfahren nach Anspruch 1, bei dem die Reinigung des Inertgasstroms durch Waschen mit einem wäßrigen Medium mit einem pH im Bereich von 7 bis 12 erfolgt.

3. Verfahren nach Anspruch 2, bei dem die Sperrflüssigkeit des zur Kompression des Inertgases eingesetzten Kompressors als Waschmedium dient.

4. Verfahren nach Anspruch 2 oder 3, bei dem der Inertgasstrom nach der alkalischen Wäsche und vor der Rückführung in Schritt a) einer zweiten Wäsche in pH-neutralem wäßrigen Medium zugeführt wird.

5. Verfahren nach Anspruch 1, bei dem die Reinigung des Inertgasstroms durch Überleiten über ein Festbettadsorbens erfolgt.

6. Verfahren nach Anspruch 5, bei dem als Festbettadsorbens Aktivkohle oder Zeolith eingesetzt wird.

## Claims

1. A process for producing bis(4-hydroxyaryl)alkanes from addition products of bis(4-hydroxyaryl)alkanes and aromatic hydroxy compounds, wherein
a) an inert gas is passed through a melt of an addition product of a bis(4-hydroxy-aryl)alkane and an aromatic hydroxy compound at 150°C to 230°C, wherein the inert gas stream removes the aromatic hydroxy compound from the melt,
b) the aromatic hydroxy compound is removed from the inert gas stream by condensation,
c) the inert gas stream is purified, compressed and recycled to step a).

2. A process according to claim 1, wherein purification of the inert gas stream is effected by scrubbing with an aqueous medium with a pH within the range from 7 to 12.

3. A process according to claim 2, wherein the sealing liquid of the compressor used for the compression of the inert gas is employed as the scrubbing medium.

4. A process according to claims 2 or 3, wherein after alkaline scrubbing and before it is recycled to step a) the inert gas stream is subjected to a second scrubbing step in an aqueous medium of neutral pH.

5. A process according to claim 1, wherein purification of the inert gas stream is effected by passing it over a fixed bed adsorbent.

6. A process according to claim 5, wherein activated carbon or zeolite is used as the fixed bed adsorbent.

## Revendications

1. Procédé de préparation de bis(4-hydroxyaryl)alcanes à partir d'adduit de bis(4-hydroxyaryl)alcanes et de composés hydroxylés aromatiques, dans lequel :
a) on fait passer un gaz inerte dans une masse fondue constituée d'un adduit d'un bis(4-hydroxyaryl)alcane et d'un composé hydroxylé aromatique de 150°C à 230°C, où le courant de gaz inerte élimine le composé hydroxylé aromatique de la masse fondue,
b) le composé hydroxylé aromatique est éliminé du courant de gaz inerte par condensation,
c) le courant de gaz inerte est purifié, comprimé et à nouveau ramené à l'étape a).

2. Procédé suivant la revendication 1, dans lequel la purification du courant de gaz inerte est réalisée par lavage avec un milieu aqueux à un pH dans l'intervalle allant de 7 à 12.

3. Procédé suivant la revendication 2, dans lequel le liquide d'obturation du compresseur mis en oeuvre pour la compression du gaz inerte sert de milieu de lavage.

4. Procédé suivant la revendication 2 ou 3, dans lequel le courant de gaz inerte est amené, après le lavage alcalin et avant le renvoi à l'étape a), dans un deuxième lavage avec un milieu aqueux à pH neutre.

5. Procédé suivant la revendication 1, dans lequel la purification du courant de gaz inerte est réalisée par passage sur un absorbant en lit fixe.

6. Procédé suivant la revendication 5, dans lequel on met en oeuvre du charbon actif ou une zéolite comme absorbant en lit fixe.
